# EUROPEAN PATENT SPECIFICATION

(11) **EP 3 003 160 B1**
(45) Date of publication and mention of the grant of the patent: **18.04.2018**
(21) Application number: 14807393.5
(22) Date of filing: 30.05.2014
(51) Int. Cl.: A61M 25/01, A61M 27/00, A61B 34/20, A61B 8/08, A61B 8/00, A61B 10/00, A61M 25/00

(54) **APPARATUS FOR POSITIONING MEDICAL DEVICE**
VORRICHTUNG ZUR POSITIONIERUNG EINER MEDIZINISCHEN VORRICHTUNG
APPAREIL DE POSITIONNEMENT D'UN DISPOSITIF MÉDICAL

(30) Priority: 04.06.2013 US 201361830758 P
(43) Date of publication of application: 13.04.2016
(73) Proprietor: Navisonics, Inc., Shoreline, Washington 98177 (US)
(72) Inventor: QUISTGAARD, Jens Ulrich, Shoreline, Washington 98177 (US)
(74) Representative: WSL Patentanwälte Partnerschaft mbB
(86) International application number: PCT/US2014/040143
(87) International publication number: WO 2014/197295

(56) References cited:
- WO-A1-2013/086521
- US-A- 5 156 157
- US-A- 5 690 117
- US-A- 5 690 117
- US-A1- 2004 267 121
- US-A1- 2005 049 510
- US-A1- 2007 083 100
- US-A1- 2008 287 916
- US-A1- 2010 256 483
- US-A1- 2012 010 506
- US-A1- 2012 010 506

## Description

The invention relates to an apparatus for positioning a medical device within or in proximity to a fluid containing compartment within a human body.

### BACKGROUND

The use of ultrasound to gather information about structure within living bodies, including the brain, has been practiced since at least the early 1940's. Ultrasound waves may be launched into tissue by an electrically stimulated transducer, which is typically constructed from a piezoelectric material. Once launched from the transducer, ultrasound waves interact with structures that they encounter, and may be transmitted, reflected, or scattered depending on the physical characteristics of the structure encountered. In particular, reflections or "echoes" arise when the ultrasound waves transition between materials (in this case, usually tissues or fluids) that have different acoustic impedances.

The use of ultrasound specifically to aid in placement of a medical device within or in proximity to a substantially fluid filled compartment within a human body is also not new. U.S. Patent No. 5,690,117, describes an ultrasound-tipped silastic intracranial catheter which also incorporates optical imaging. Standard diagnostic ultrasound systems are used routinely to aid in cannulization of blood vessels, lancing of cysts, placement of drain catheters, and so forth.

The use of echolocation where echoes of sound waves reflect from remote structures over time scales that are perceptible to a human is also not new. The earliest SONAR systems consisted simply of a sound transducer, amplifier, and speaker, where a sound wave was launched into water from the transducer, and then the operator would listen to amplified signals from the transducer for echoes. The time it would take for an echo to return to the transducer gave an indication of the distance to the reflecting structure. This sort of technique is not directly usable in medical ultrasound because the distances are small (usually less than 10cm), and the echoes return so quickly that they are not evident to unaided human perception.

The use of echolocation or RADAR to determine the position of structures, and then coding that information as audio information that is "time stretched" is also not new. For example, many modern automobiles incorporate hazard sensors where echolocation or RADAR is used to detect obstacles, and feedback is given to the driver as a series of audio "beeps" - the closer the obstacle is, the faster the beeps occur, leading up to a continuous tone when the obstacle is within a pre-determined distance from the vehicle. In this case, while the difference in echo time between the vehicle and obstacles at different distances may be on the order of milliseconds, the period between beeps indicating distance may be on the order of a second, which is easily perceptible by a human. Thus, the echo time is "stretched" so as to be perceptible.

In medicine, there is often a need to quickly and accurately place a medical device within or in proximity to a substantially fluid filled compartment within a human body. For example, it may be necessary to place a catheter in a ventricle of the brain to allow drainage of cerebrospinal fluid. Such a device is for example described in US 5,690,117, wherein ultrasound imaging is performed to visualise the ventricular system of the brain.

### BRIEF SUMMARY OF THE INVENTION

To address the above mentioned problems and others, the technology disclosed herein couples an ultrasound echo-location device with "time stretching" techniques to provide a simple and effective form of guidance for the placement of a medical device within or in proximity to a substantially fluid filled compartment within a human body. The apparatus constructed in accordance with an embodiment of the invention utilizes a rigid or minimally flexible hollow member with an ultrasound transducer mounted at its tip situated within an intracranial catheter to allow echolocation through the tip of the catheter. The ultrasound transducer is electrically coupled to both transmit and receive electronics via an electrical connection that may involve wires, the hollow member itself, or both the hollow member and one or more wires as conductors. Transmit electronics are provided to electrically excite the transducer so acoustic waves are generated. Receive electronics are provided to electrically amplify and process signals from the transducer that arise due to returning echoes from the transmitted waves. Processing of the received signals includes the derivation of a time-stretched signal that is representative of the originally received signal, but with features of the signal spread out over a time interval so that they are perceptible to a human. One or more audio, visual, or tactile outputs are provided in order to indicate the presence of a substantially fluid filled space, and give a qualitative or quantitative indication of the distance from the ultrasound transducer to this space.

### BRIEF DESCRIPTION OF THE DRAWINGS

Fig. 1 schematically illustrates a system according to an embodiment of the invention.
Fig. 1a illustrates a returning echo signal with a fluid-filled space present.
Fig. 1b illustrates an algorithm to determine whether or not a fluid filled space is present, and to determine the location of the space.
Fig. 1c illustrates an algorithm to compute a "stretched" time corresponding to the middle of a fluid filled space.
Fig. 1d illustrates an algorithm to track the position of an ultrasound transducer with respect to a fluid filled space in the presence of ring-down or other initial noise signals.
Fig. 2 schematically illustrates the signal acquisition and transmission portion of a wirelessly coupled system according to an embodiment of the invention.
Fig. 3 schematically illustrates the signal processing, control, and user interface portion of a wirelessly coupled system according to an embodiment of the invention.
Fig. 4 illustrates an embodiment of this invention where all circuitry including a power source and user interface is incorporated in a housing that is directly coupled to a hollow member incorporating an ultrasound transducer. The hollow member is shown inserted into a catheter.
Fig. 5 illustrates an embodiment of this invention where the hollow member incorporating the ultrasound transducer is coupled to circuitry including a power source and user interface through a flexible cable.
Fig. 5a illustrates an embodiment of this invention where the hollow member incorporating the ultrasound transducer is coupled directly to a portion of circuitry which may include a power source and user interface, which is then coupled to additional circuitry which may contain a power source and user interface through a flexible cable.
Fig. 6 shows a detail of the distal end of the hollow member incorporating an ultrasound transducer inserted into a catheter intended for drainage of cerebrospinal fluid which incorporates a feature that allows the hollow member to be pushed through the tip of the catheter.
Fig. 7 shows a detail of the distal end of the hollow member incorporating an ultrasound transducer inserted into a catheter intended for drainage of cerebrospinal fluid with the hollow member incorporating an ultrasound transducer is pushed through the tip of the catheter.
Fig. 8 shows a detail of the distal end of the hollow member incorporating an ultrasound transducer inserted into a catheter intended for drainage of cerebrospinal fluid which is open at the distal end allowing an unimpeded acoustic path to the front of the ultrasound transducer.
Fig. 9 shows a detail of the distal end of the hollow member incorporating an ultrasound transducer inserted into a catheter intended for drainage of cerebrospinal fluid which is open at the distal end allowing an unimpeded acoustic path to the front of the ultrasound transducer. The tip of the catheter is designed so that a lip exists inside the tip of the catheter. The hollow member is designed so that a portion of it can push against the lip in the catheter during insertion, while still allowing the ultrasound transducer to be positioned in the open tip of the catheter.
Figure 10 shows a detail of an embodiment of the distal end of the hollow member incorporating an ultrasound transducer with electrical connections for the transducer and a protective coating.
Figure 11 shows a detail of another embodiment of the distal end of the hollow member incorporating an ultrasound transducer with electrical connections for the transducer and a protective coating.

### DETAILED DESCRIPTION OF THE INVENTION

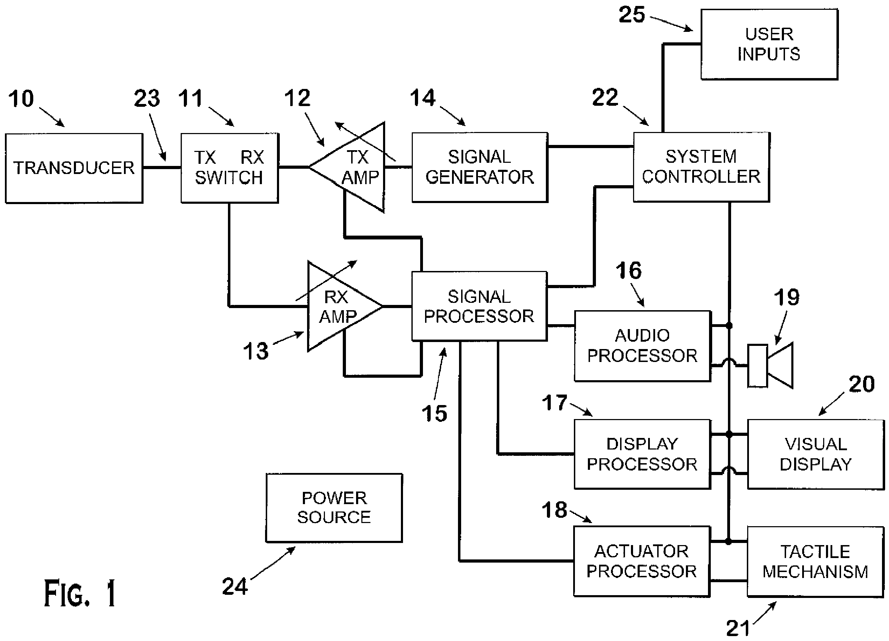

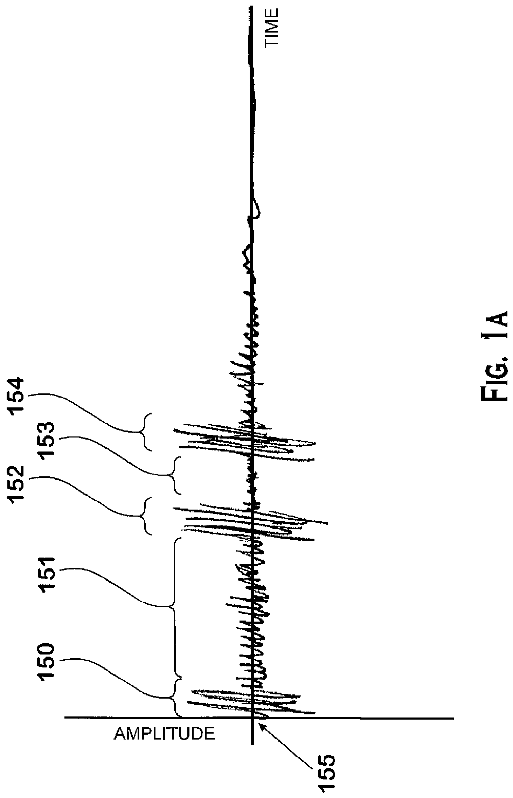

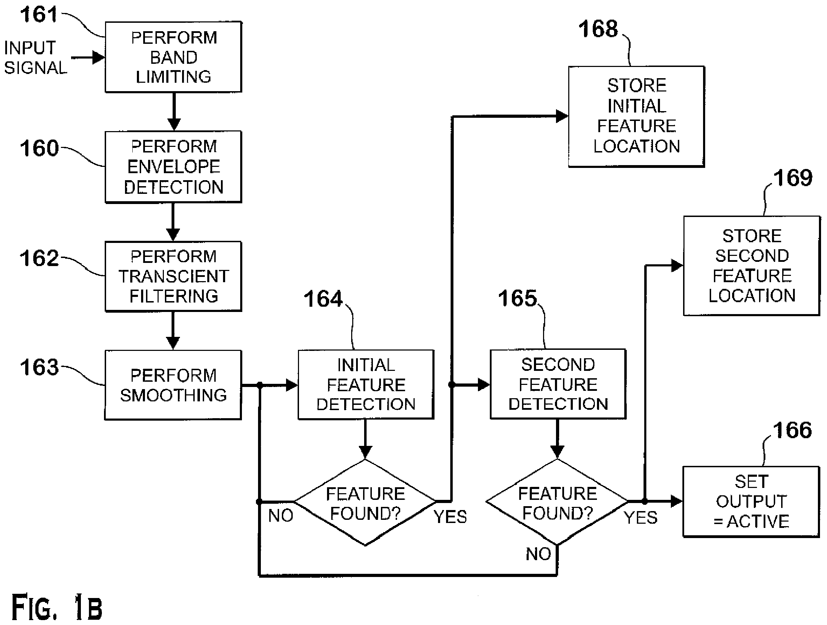

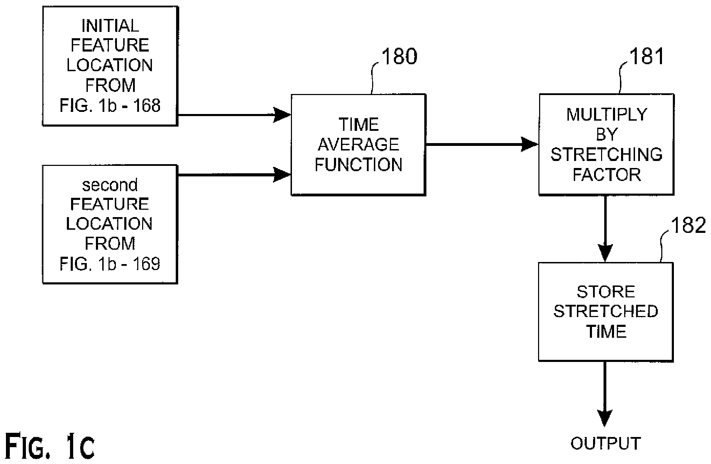

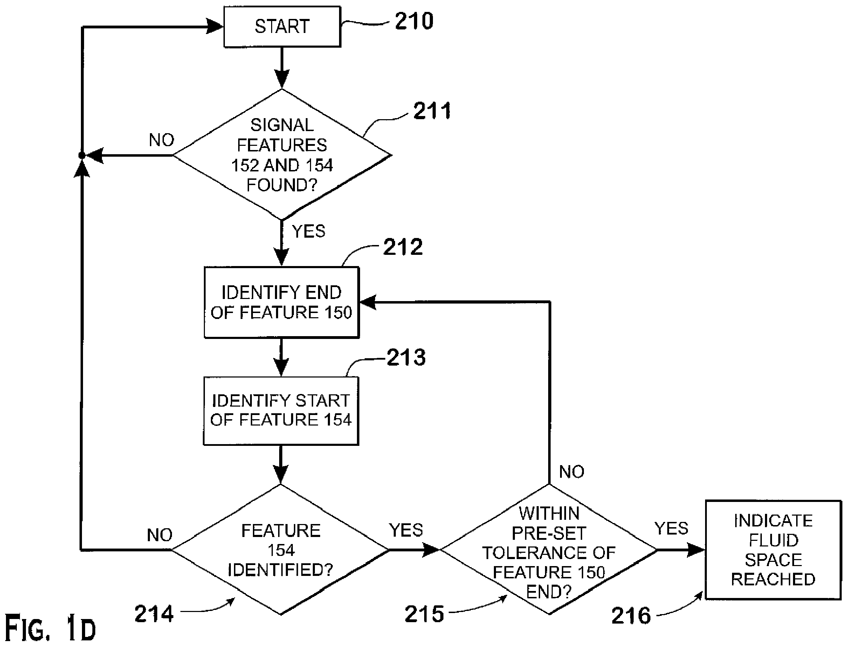

Referring now to Fig. 1 there is shown an ultrasound transducer 10 coupled to transmit / receive switch circuit 11. In this embodiment, transmit / receive circuit 11 is shown without control connections to either signal processor 15 or system controller 22, though such control connections may be incorporated in other embodiments. A signal generator 14 is connected to transmit amplifier 12. In this embodiment, transmit amplifier 12 is shown as a variable gain amplifier with the gain being controlled by signal processor 15, though in other embodiments amplifier 12 may be a fixed gain amplifier, or may be controlled by system controller 22. The transmit amplifier 12 is connected to the transmit port of the transmit / receive switch circuit 11. The receive port of the transmit / receive switch circuit 11 is connected to receive amplifier 13 which Is connected to signal processor 15. In this embodiment, receive amplifier 13 is shown as a variable gain amplifier with the gain being controlled by signal processor 15, though in other embodiments amplifier 13 may be a fixed gain amplifier, or may be controlled by system controller 22.

Signal processor 15 is connected to audio processor 16, display processor 17, and tactile processor 18, all of which are intended to drive elements of a user interface providing information to a user. Audio processor 16 is connected to audio transducer 19 which can produce audible information. Display processor 17 is connected to visual display 20, which can produce visual information. Tactile processor 18 is connected to tactile mechanism 21, which can produce information that can be sensed by touch.

In a preferred embodiment, audio transducer 19 is a piezoelectric transducer. In another embodiment, audio transducer 19 may be an electromagnetic speaker. It will be appreciated that many different audio transducer technologies may be utilized to fulfill the intent of the present invention.

In a preferred embodiment, visual display 20 is made up of one or more light emitting diodes (LEDs). In one such embodiment, a single LED may be used to indicate the presence or absence of fluid and / or distance to the fluid, to the user through modulation of the brightness of the emitted light. In another such embodiment, two or more LEDs may be used to indicate the presence or absence of fluid to the user through modulation of the combined color of the light emitted by the LEDs. In another such embodiment, two or more LEDs may be used as an indicator of fluid through modulation of both brightness and color. In another such embodiment, many LEDs may be arranged so that an image may be formed which conveys information regarding the presence or absence of fluid. In such an embodiment, the image may be textual, graphical, or both. In a further embodiment, visual display 20 is made up of one or more liquid crystal displays. It will be appreciated that many different display technologies may be utilized to fulfill the intent of the present invention.

In a preferred embodiment, tactile mechanism 21 is an electromechanical vibration device, such as an asymmetrical weight attached to an electric motor. In one such embodiment, the speed of the vibration may be used to indicate the presence or absence of fluid and / or distance to the fluid. In another such embodiment, the intensity of the vibration may be used as an indicator. In another such embodiment, the speed or intensity of vibration may be varied over time as an indicator. As an example of such an embodiment, the presence or absence of vibration indicates the presence or absence of fluid in front of the ultrasound transducer, while the time between short bursts of vibration indicates distance to the fluid from the ultrasound transducer face. In another embodiment, a solenoid may be used to move a mechanism to provide tactile feedback. It will be appreciated that many different tactile feedback technologies, including haptic technologies, may be utilized to fulfill the intent of the present invention.

Audio processor 16, display processor 17, tactile processor 18, visual display 20 and tactile mechanism 21 are shown as connected to system controller 22. In any particular embodiment of the present invention, these connections may or may not be required.

User inputs 25 provide means for the user to enable, disable, configure, or provide other input to the system. In a preferred embodiment, user inputs 25 incorporate electromechanical switches. In other embodiments, user inputs 25 may incorporate potentiometers, rotary encoders, or proximity sensors. It will be appreciated that many different input technologies may be utilized to fulfill the intent of the present invention.

Power source 24 supplies power to elements which require it. The power source is preferably a rechargeable battery, though it may be a non-rechargeable battery, another electrical storage device such as a capacitor, or a power supply drawing energy from mains or an electrical energy generation apparatus.

In an embodiment, system controller 22 triggers signal generator 14 to produce an electrical waveform that is amplified by transmit amplifier 12, passes through transmit / receive switch circuit 11, and excites transducer 10 so that it vibrates and produces acoustic waves that travel through tissue that is in contact with the transducer. Transmit / receive switch circuit 11 substantially blocks the amplified transmit signal from entering receive amplifier 13. The transmit / receive switch circuit may be a diode-based directional circuit which is well understood and has been used in diagnostic ultrasound systems for many years. Alternatively, the switch circuit may be a solid-state switch which is controlled by either the system controller 22 or the signal processor 15. The frequency of the acoustic waves produced in the system may vary greatly, but will preferably be in the range of 1 to 20 MHz. The number of wave cycles transmitted in a pulse may also vary greatly, but will preferably be in the range of 1 to 20 cycles. The pulse repetition frequency of the system may also vary greatly, but will preferably be in the range of 1 to 20000 Hz.

Echoes returning to the transducer 10 from the tissue coupled to the transducer are converted to an electrical waveform by the transducer 10 and passed through the transmit / receive switch circuit 11 to the receive amplifier 13. The amplified signal is then passed to signal processor 15 to be analyzed. In a preferred embodiment, signal processor 15 is a digital signal processor, incorporating an analog to digital converter so that the output of the receive amplifier 13 may be converted to digital form. In another embodiment, signal processor 15 is an analog signal processor, which may be as simple as a threshold detector that determines whether or not the amplitude of the received signal has exceeded a predetermined or adaptive limit. In a preferred embodiment, signal processor 15 controls the gain of the receive amplifier 13 in order to implement time-gain compensation (TGC). TGC is a well understood concept that has been incorporated in diagnostic ultrasound systems for many years, where gain is increased over time to compensate for the loss of energy in acoustic waves as they travel longer distances through tissue or fluid.

The signal processor 15 is responsible for analyzing the signal representing returning echoes primarily to determine whether a fluid filled space is present in front of the transducer 10, and if so, to convert this information to a form more suitable for feedback to the user. Fig. 1 a illustrates the time-amplitude properties of a typical signal indicating the presence of a fluid-filled space. It will be appreciated that in such an illustration, time also represents distance traveled by an acoustic wave. An assumption is generally made for the speed of sound in tissue, which is about 1540 meters per second. The signal begins at point 155, which corresponds to the time when the system excites the ultrasound transducer to create the acoustic waves that will be used to search for a fluid filled space. A strong initial signal 150 may be present due to effects such as ring-down of the transducer. This initial signal is not relevant to fluid detection and should be minimized. The next portion of the signal 151 represents low level echoes from features within the tissue. Echo feature 152 is a higher amplitude feature representing the transition from tissue to fluid, while the next portion of the signal 153 is essentially echo free and represents the fluid. Echo feature 154 is a higher amplitude feature representing the transition from fluid to tissue, while the next portion of the signal 155 represents additional echoes from tissue features beyond the fluid compartment. The combination of features 152, 153, and 154 form the signature of a fluid filled space, that is, an initial echo feature indicating transition from tissue to fluid followed by a relatively echo free region, followed by a trailing echo feature indicating transition from fluid to tissue.

Because the signal features 152 and 154 are generally distinguished by their relatively larger magnitude from surrounding features in the echo signal, an algorithm may be implemented to identify the fluid space automatically. Fig. 1b illustrates such an algorithm. In this embodiment, the algorithm is implemented by first setting a binary detection signal 166 to its inactive state, and then performing envelope detection 160 on the echo signal to yield a relatively smoothed version of the returning echo signal. A filtering operation may be added before or after envelope detection in order to improve the signal's suitability for analysis. In a preferred embodiment, a band limiting filter 161 is provided before the envelope detection, and a transient filter 162 followed by a smoothing filter 163 is provided after envelope detection. It will be appreciated that such filters may implement many different types of processing. Processing block 164 attempts to identify an initial signal feature corresponding to signal feature 152 by examining portions of the echo signal in sequence from its beginning until a feature is found with amplitude significantly greater than its surrounding regions, and with duration consistent with a tissue to fluid interface. In one embodiment, such a feature is considered to be found if its amplitude exceeds twice the amplitude of the surrounding signal regions, and its duration exceeds the pulse length of the ultrasound signal used to interrogate the tissue and fluid. If this signal feature is found, processing block 165 attempts to identify a subsequent signal feature corresponding to feature 154 by examining further portions of the echo signal in sequence until a feature is found with amplitude significantly greater than its surrounding regions, and with duration consistent with a tissue to fluid interface. If this pattern of signal features is found, a fluid filled space is considered to have been found and a binary detection signal 166 is turned to its active state.

It will be appreciated that knowledge of anatomy relevant to a specific application may be applied to improve detection performance. For example, in an application where the present invention is being used to guide the placement of a ventricular drainage catheter, it is known that in the vast majority of cases the ventricle will not be found in the first two centimeters of tissue, nor will it be found deeper than 8 centimeters from the brain surface. Therefore, in one embodiment, signal features indicating fluid spaces outside these distances are ignored for detection purposes.

If a fluid filled space is detected, its approximate distance from the face of the ultrasound transducer and its approximate extent in the direction of the ultrasound beam is easily determined by computing the time intervals between the initial ultrasound pulse and the returning echoes from signal features corresponding to features 152 and 154 in Fig. 1a. The time points representing features 152 and 154 are preferably the midpoint of their extent in time, but may also be chosen to be anywhere within, immediately before, or immediately after their extent in time. Once these three time points are determined, the current position of the ultrasound transducer being used to guide the medical device relative to the fluid filled space may be determined and used to provide feedback to a user. Processing blocks 164 and 165 determine these two time points, store them, and make them available for subsequent processing through outputs 168 and 169. In a practical implementation, the outputs 168 and 169 may represent time in any suitable scale, for example, samples in a digital system, or as a number representing microseconds. The algorithm illustrated in Fig. 1b thus provides an indication as to whether or not a fluid filled space is present in front of the ultrasound transducer, and if so, at what depth in front of the transducer the leading and trailing edge of this space lies.

Detection of a fluid-filled space can happen within tens of microseconds. If we assume that ultrasound travels through tissue at 1540 meters per second, or approximately 1.54 millimeters per microsecond, then a fluid filled space 4cm from the ultrasound transducer would give an initial echo corresponding to feature 152 in Fig. 1a in about 52 microseconds. In a case where the extent of the fluid filled space in the direction of the ultrasound beam is about 1cm, the echo corresponding to feature 154 in Fig. 1a would arrive about 12 microseconds later, depending on the speed of sound in the fluid. Events occurring in such short timeframes are generally not perceptible by human, so some mechanism must be employed to spread their presentation out over time, space, or both so as to make them perceptible. A simple example of this is the oscilloscope, which measures an electrical waveform that may have megahertz or gigahertz bandwidth, but then allows the user to examine details of the signal on a display where microseconds or nanoseconds of signal information are spread out over a display and held for a time so that the signal information is perceptible. Oscilloscopes were used in early diagnostic ultrasound systems, and in fact could be used to produce displays similar to that shown in Fig. 1a. Ultrasound imaging systems generally encode signal amplitude as brightness and time as distance in a display. A series of echo signals are processed and accumulated to produce a single image.

Previous investigators have used oscilloscopes or oscilloscope-like displays to present information similar to that shown in Fig. 1a to aid in finding fluid filled spaces within tissue for many years. Recently, this technique has been demonstrated for the detection of cerebrospinal fluid in the brain, and proposed to aid the correct placement of a ventricular drainage catheter in the brain. A limitation of this technique is that it requires a user to interpret this oscilloscope-like display, which may not be intuitive. It is an object of the present invention to overcome this limitation by simplifying the presentation of echo information so that it is more easily understood, specifically by identifying fluid filled spaces automatically, using time-stretching techniques to present information to a user in an easier to understand format, using audio, visual, and tactile feedback, either singly or in combination.

Fig. 1c illustrates an algorithm for performing the above described time stretching operation utilizing the outputs of the algorithm of Fig. 1b. First, processing block 180 computes a time point corresponding to the distance half way between the leading edge of the fluid filled space and the trailing edge of the fluid filled space using outputs 168 and 169. This represents the approximate time that it would take an acoustic wave to travel from the transducer to the middle of the fluid filled space and back to the transducer. Multiplier 181 then multiplies this time by a stretching factor that will make this time perceptible to a human. In a preferred implementation, this stretching factor is chosen to be about 20000, so that a time of 50 microseconds is stretched out to one second. Output 182 represents this "stretched" time.

Referring now again to Fig. 1, and understanding that the algorithms described above and illustrated in Fig. 1b and Fig. 1c are implemented by signal processor 15, the outputs 166 and 182 are provided to audio processor 16, display processor 17, and actuator processor 18. In a preferred embodiment, audio processor 16 generates an electrical signal representing a short audible tone or "beep" which repeats at the temporal rate indicated by output 182 if output 166 is active, otherwise no signal is generated. This electrical signal is then used to generate a series of audible beeps through audio transducer 19. In this preferred embodiment, display processor 17 generates an electrical signal suitable for driving a LED so that it flashes at the temporal rate indicated by output 182 if output 166 is active, otherwise no signal is generated. This electrical signal is then used to drive visual display 20, which in this case is simply a LED, so that it flashes as described above. In this preferred embodiment, actuator processor 18 generates an electrical signal suitable for driving a solenoid with a spring-return plunger of sufficient mass so that its motion is perceptible by touch in a pulsed fashion at a temporal rate indicated by output 182 if output 166 is active, otherwise no signal is generated. This electrical signal is then used to drive tactile mechanism 21, which in this case is simply the solenoid described above. Thus, in this embodiment, if a fluid filled space is present so that its center is about 4 centimeters in front of the ultrasound transducer 10, audio transducer 19 will beep at 1Hz, visual display 20 will flash at 1Hz, and tactile mechanism 21 will make a physical movement perceptible by touch at 1Hz. As the ultrasound transducer 10 is moved closer to the fluid filled space, the rate of audio, visual, and tactile feedback will increase.

It will be appreciated that any of the audio, visual, or tactile feedback mechanisms may be enabled or disabled in any combination by the system controller 22. It will also be appreciated that, in any particular implementation, mechanisms for audio, visual, or tactile feedback may or may not be present, so long as at least one such feedback mechanism is present. It will also be appreciated that, in any particular implementation, mechanisms for audio, visual, or tactile feedback may be used to provide different types of feedback at the same time. In one embodiment, for example, visual display 20 may display a red light if a fluid filled space is not detected and a green light if such a space is detected, while audio processor and transducer 16 and 19 provide a series of beeps indicating distance to the fluid filled space if such a space is detected, and tactile mechanism 21 provides feedback through physical motion when the ultrasound transducer is determined to be in the fluid filled space. It will be apparent that the mechanisms for audio, visual, and tactile feedback may be used many such combinations to provide feedback to the user.

It will be appreciated that, as the ultrasound transducer 10 is moved closer to the fluid filled space, at some point the signal feature 152 will blend with signal feature 150 and may become indistinguishable therefrom. If the ultrasound transducer is moved through the fluid filled space, ultimately signal feature 154 will blend with signal feature 150 and may become indistinguishable therefrom. Fig. 1d further illustrates how this situation may be addressed in practice.

In Fig. 1d, features corresponding to the signal features identified in Fig. 1a are referenced. The algorithm is initialized in box 210, which clears any indication that a fluid filled space has been reached and clears any stored location in processing block 213. Decision block 211 indicates whether signal features 152 and 154 of Fig. 1a have been found as described herein, indicating the presence of a fluid filled space. If these signal features have not been found, the algorithm returns to its initialization state 210. If the signal features have been found, processing block 212 determines the end of signal feature 150. In an embodiment, this determination may be made by a threshold test after block 163 of Fig. 1b. Processing block 213 determines the beginning of signal feature 154. In an embodiment, this determination may be made by a threshold test as may be performed in block 164 of Fig. 1b. Processing block 213 then compares the location of signal feature 154 to any previously stored location. If the current location is within a pre-determined distance from the previously stored location, tracking is considered successful and feature 154 is indicated to have been identified. If the stored location information is cleared, as would be the case in the first iteration through the algorithm, feature 154 would also be indicated as having been found. If the location of signal feature 154 is outside of the pre-determined distance from the previously stored location, tracking is considered unsuccessful, and feature 154 is indicated as not having been identified. Decision block 214 determines whether or not the start of signal feature 154 has been found in processing block 213. If it has not been found, tracking is lost and the algorithm returns to its initialization state 210. If it has been found, decision block 215 determines whether the beginning of signal feature 154 is within a pre-set tolerance of the end of signal feature 150. If it is, an indication is given to the user that the fluid-filled space has been reached. If not, the algorithm returns to processing block 154.

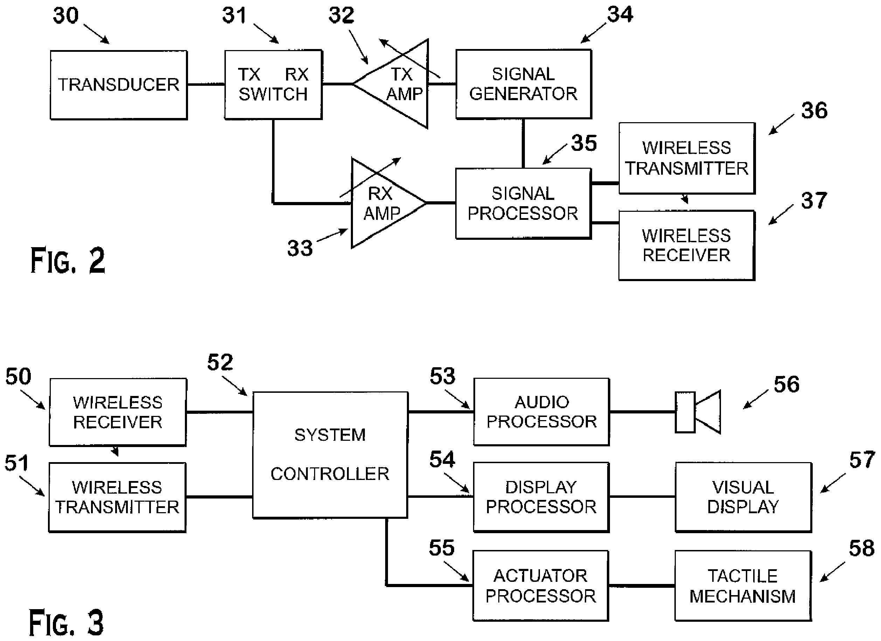

Fig. 2 is a block diagram of a portion of the system illustrated in Fig. 1, but with a wireless transmitter 36 and receiver 37 added. Fig. 3 shows the remainder of the elements of Fig. 1 that are missing from Fig. 2, with the addition of wireless receiver 50 and transmitter 51. The combination of the systems illustrated in Fig. 2 and Fig. 3 thus, in combination, may implement all of the functions discussed above for the system illustrated in Fig. 1 by sending appropriate data across a wireless link formed with the wireless transmitters and receivers. In a preferred implementation, the wireless link is formed with low-power Bluetooth™ components, though it will be appreciated that many different forms of communication, including RF, optical, and ultrasonic means, may be used to form this link. It will also be appreciated that certain modifications to the architecture of Fig. 1 may be made to most efficiently incorporate such a wireless link, for example, control over signal generator 14 by system controller 22 in Fig. 1 may be implemented by passing such control information from system controller 52 wirelessly to signal processor 35 and then on to signal processor 34. It will also be appreciated that the wireless link described above may be placed at many different locations in the architecture of Fig. 1, for example in another embodiment the wireless transmitter 36 may be connected directly to receive amplifier 35 and the signal processing function would be performed on the other side of the wireless link. In such an implementation, wireless receiver 36 may be connected directly to signal generator 34 so as to provide a means for triggering transmission of ultrasound pulses from the system controller 52. It will also be appreciated that a similar implementation may incorporate certain signal processing functions on both sides of the wireless link, for example the band limiting filter function may be implemented in signal processor 35 of Fig. 2, while an additional signal processor added to the system illustrated in Fig. 3 may implement detection and smoothing operations.

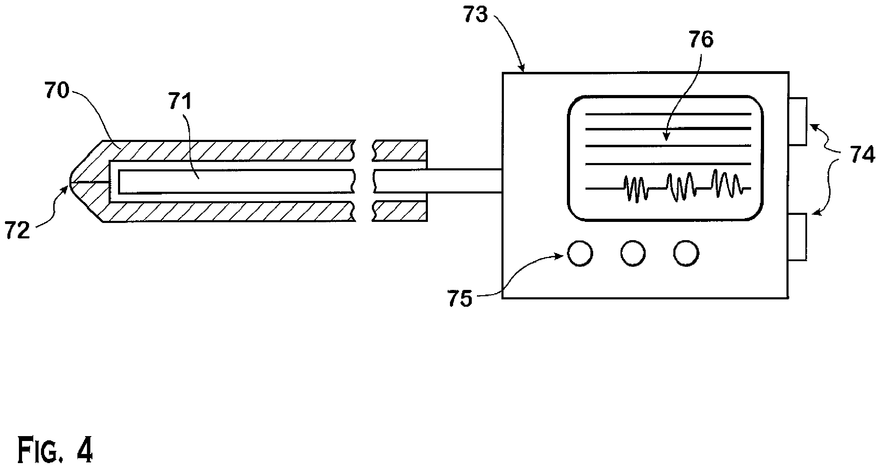

The present invention is particularly suitable for guiding the placement of a catheter to drain cerebrospinal fluid from one of the ventricles of the brain. Fig. 4 illustrates a physical implementation of the system illustrated in Fig. 1. Hollow member, or "stylet" 71 with an ultrasound transducer corresponding to transducer 10 of Fig. 1 positioned at its distal end is shown inside a cut-away view of drainage catheter 70. The drainage catheter will incorporate at least one drainage port in the side of the catheter near its distal end, but such ports are not shown in the illustration. The tip of drainage catheter 70 incorporates a "fishmouth" slit 72 that allows stylet 71 to be pushed through the otherwise closed end of the catheter 70 as is described further below. Stylet 71, which is preferably constructed of an electrically conductive material and coupled to the face of the ultrasound transducer, also contains a wire connected to the rear of the ultrasound transducer, which in combination with the conductive stylet form an electrical circuit corresponding to connection 23 in Fig. 1. Stylet 71 is mechanically coupled to housing 73, which contains electronics and a power source corresponding to the other elements in Fig. 1. In this embodiment, the power source is preferably a battery power source, though it may be another electrical storage device such as a capacitor, or a power supply drawing energy from mains or an electrical energy generation apparatus. Graphical display 76 and LED lamps 75 are shown, both corresponding to visual display 20 of Fig. 1, as are user inputs 74 corresponding to user inputs 25 of Fig. 1.

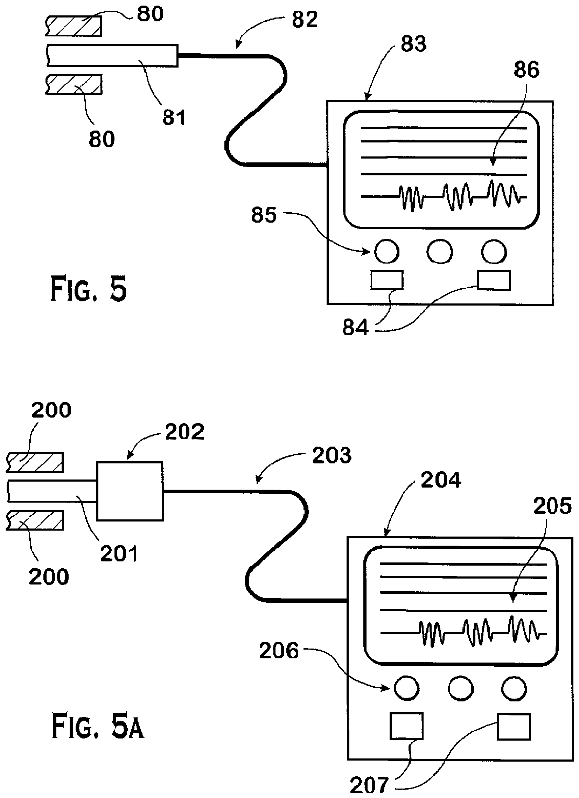

Fig. 5 illustrates a similar embodiment to that of Fig. 4, where now the stylet 81 corresponding to stylet 71 of Fig. 4 is connected by a small co-axial cable 82 to housing 83 which contains the electronics and power source. In this embodiment, the power source is preferably a rechargeable battery, though it may be a non-rechargeable battery, another electrical storage device such as a capacitor, or a power supply drawing energy from mains or an electrical energy generation apparatus. The proximal end of the catheter 80 corresponding to the catheter 70 of Fig. 4 is shown, as are the graphical display 86 and LED lamps 85 corresponding to elements 76 and 75, respectively, of Fig. 4. User inputs 84 corresponding to user inputs 74 of Fig. 4 are also shown.

Fig. 5a illustrates a similar embodiment to that of Fig. 4, where now the stylet 201 corresponding to stylet 71 of Fig. 4 is mechanically coupled to housing 202 containing electronics, which is in turn connected by cable 203 to housing 204 which contains electronics. It will be appreciated that housing 202 may actually be the same as hollow stylet 201. In an embodiment, electrical tuning components such as one or more inductors or capacitors may be incorporated in close physical proximity to the transducer at the distal end of hollow stylet 201. It will be appreciated that a power source may be incorporated in housing 202, housing 204, or in both. Preferably, a power source is incorporated in housing 204, and any power required by electronics contained in housing 202 is supplied through cable 203. In this embodiment, the power source is preferably a rechargeable battery, though it may be a non-rechargeable battery, another electrical storage device such as a capacitor, or a power supply drawing energy from mains or an electrical energy generation apparatus. The proximal end of the catheter 200 corresponding to the catheter 70 of Fig. 4 is shown, as are the graphical display 205 and LED lamps 206 corresponding to elements 76 and 75, respectively, of Fig. 4. User inputs 207 corresponding to user inputs 74 of Fig. 4 are also shown.

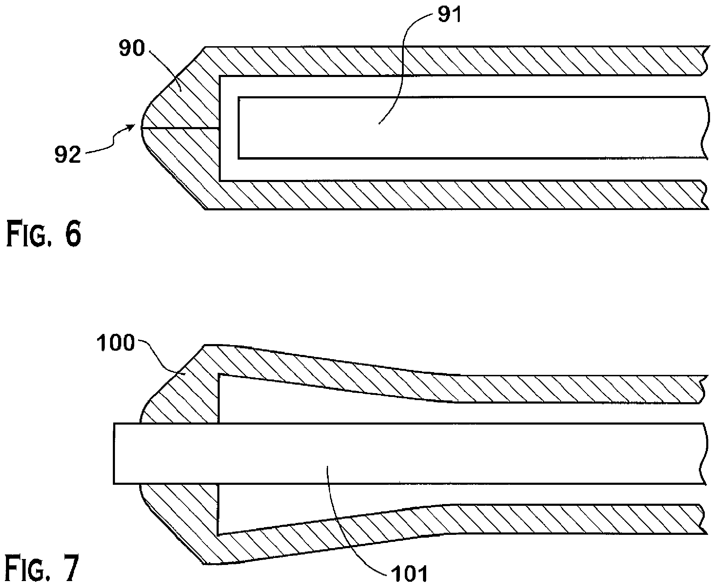

Figures 6 and 7 further illustrate the configuration of the catheter that allows the distal end of the stylet to be pushed through its end. In Fig. 6, stylet 91 incorporating the ultrasound transducer at its distal end is shown inside a cut-away view of a drainage catheter. As above, the drainage catheter will incorporate at least one drainage port in the side of the catheter near its distal end, but such ports are not shown in the illustration. A slit 92 is cut in the distal end of the catheter so that an opening may be created. In Fig. 7, the stylet 101 is now shown pushed through the slit at the distal end of the catheter 100.

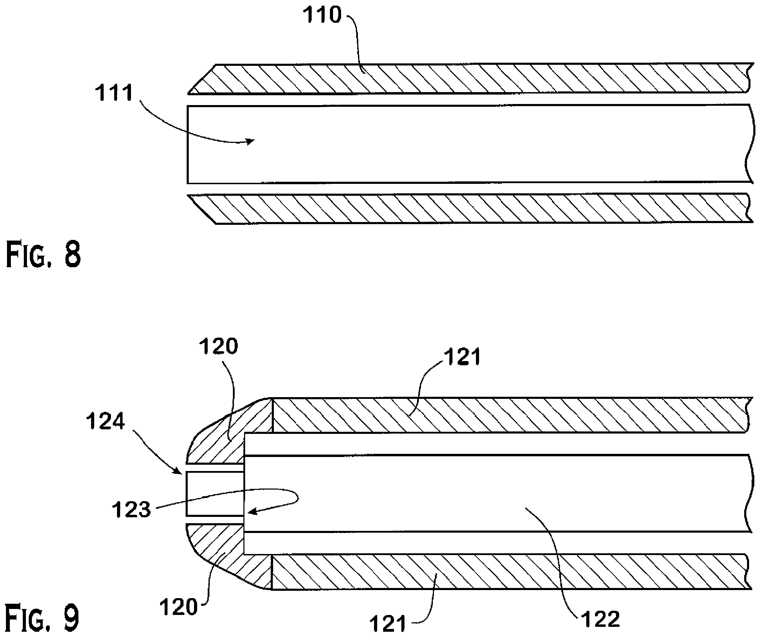

It is possible to construct a catheter such as is illustrated in Fig. 8, where the distal end of the stylet 111 is always exposed through the open end of the catheter 110. Such an implementation may have a problem with the catheter "riding up" on the stylet as it is inserted into the brain tissue. This problem may be addressed through an embodiment illustrated in Fig. 9. Here, stylet 122 incorporates a feature 123 so that its lateral dimension, or most commonly its diameter, is reduced near its distal end. This feature fits against a corresponding "lip" feature in the distal end of the catheter 120 so that the distal end of the stylet incorporating the ultrasound transducer 124 is exposed, but force may be applied to the catheter through the stylet. Preferably, the distal end of the catheter 120 is made from a material that is more rigid than the material of the body of the catheter 121.

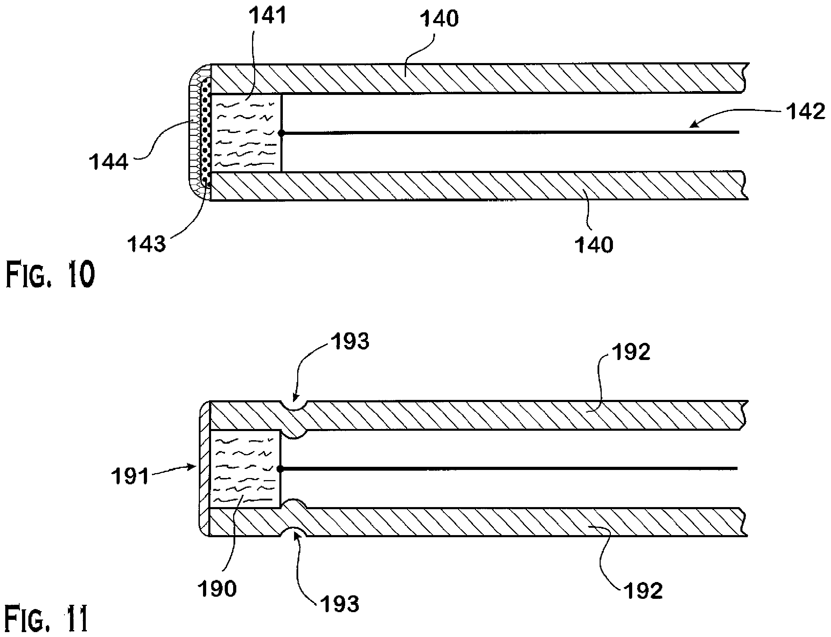

Fig. 11 illustrates a construction detail of an embodiment of the ultrasound transducer 141 corresponding to transducer 10 of Fig. 1 inside the stylet 140 corresponding to stylet 71 of Fig. 4. Here, the ultrasound transducer is most preferably constructed of a piezo-electric ceramic material, and is plated with a conductive metal, preferably silver or gold, on its front and back face. A wire 142 is bonded to the plated back face of the transducer, and the transducer is bonded inside the hollow stylet 140 with a glue. The plating on the back face of the transducer is configured so that it does not reach the edge of the transducer, so that it is not in electrical contact with the stylet. A small piece of conductive material 143 is bonded to the face of the transducer 141 and to the distal end of the stylet 140 so that the front face of the transducer is in electrical contact with the stylet. An acoustically transmissive protective coating 144, which is preferably a latex or epoxy, is deposited at the tip of the stylet. It will be apparent that the conductive material 143 is not required if the plating of the front face of the transducer wraps around the edge of the transducer so that it is in electrical contact with the stylet. Thus, the ultrasound transducer 140 may be grounded through the conductive stylet 140, and transmit and receive signals may be conveyed through wire 142.

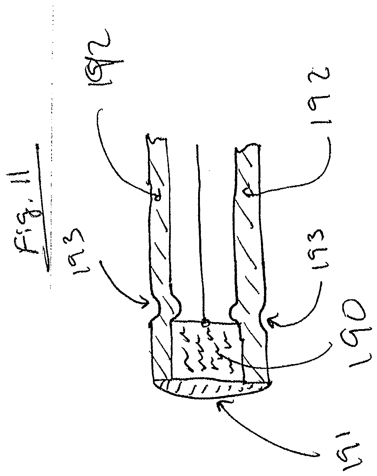

Fig. 12 also illustrates a construction detail of an embodiment of the ultrasound transducer 190 corresponding to transducer 10 of Fig. 1 inside the stylet 192 corresponding to stylet 71 of Fig. 4. Here, the plating on the front of the transducer wraps around the side of the transducer so that the additional conductive element is not required between the face of the transducer and protective layer 191. An additional feature 193 is added to the hollow stylet which is a narrowing of the stylet near its distal end that acts as a back-stop for the transducer 190 to aid in positioning during manufacturing.

## Claims

1. A surgical apparatus, comprising:
a stylet having a proximal end and a distal end and provided with a longitudinal bore therethrough;
an ultrasound transducer disposed at the distal end of said longitudinal bore of said stylet; and
electronic circuitry coupled to said ultrasound transducer;
wherein said electronic circuitry is configured to implement signal processing that detects a fluid filled space in front of said ultrasound transducer,
**characterised in that**
said electronic circuitry is further configured to implement a time stretching technique that formats information from echoes returning to the ultrasound transducer for presentation to a user.

2. The apparatus of claim 1, further comprising a visual display.

3. The apparatus of claim 1, where at least a portion of said electronic circuitry is disposed within the longitudinal bore of said stylet.

4. The apparatus of claim 1, where at least a portion of said electronic circuitry is contained in a housing that is connectably linked to said ultrasound transducer through a flexible cable.

5. The apparatus of claim 1, where said electronic circuitry is implemented in two portions which are connectably linked through a flexible cable.

6. The apparatus of claim 1, where a first portion of said electronic circuitry is disposed within a first housing that is mechanically coupled to said stylet, and a second portion of said electronic circuitry is disposed within a second housing and is connectably linked to the first portion through a flexible cable.

7. The apparatus of claim 1, where a first portion of said electronic circuitry is disposed within a first housing that is mechanically coupled to said stylet, and a second portion of said electronic circuitry is disposed within a second housing and is linked to the first portion through a wireless interface.

8. The apparatus of claim 1, where a visual display is disposed within a housing that is mechanically coupled to said stylet.

9. The apparatus of claim 4, where said stylet and cable are configured as single-use parts, and said electronic circuitry and housing are configured as multiple-use parts.

10. The apparatus of claim 1, further comprising a catheter having a proximal end and a distal end provided with a longitudinal bore therethrough and said stylet being sized and adapted for selective disposal within said longitudinal bore of said catheter.

11. The apparatus of claim 1, where said ultrasound transducer is disposed within the longitudinal bore of said stylet.

12. The apparatus of claim 1, where said ultrasound transducer is disposed outside of the longitudinal bore of said stylet.

13. The apparatus of claim 10, where said catheter incorporates an internal lip against which at least a portion of said stylet is pressed.

14. The apparatus of claim10, where said catheter incorporates an open distal end.

15. The apparatus of claim 10, where said catheter incorporates at least one drain port in a side of the cathether.

16. The apparatus of claim 1, where said stylet is composed of an electrically conductive material, and is used to form a portion of an electrical connection to said ultrasound transducer.

## Patentansprüche

1. Chirurgische Vorrichtung, welche aufweist:
Eine Sonde mit einem proximalen Ende und einem distalen Ende und mit einer Längsbohrung hierdurch,
einen Ultraschallwandler, welcher an dem distalen Ende der Längsbohrung der Sonde angeordnet ist, und
einer Elektronikschaltung, welche an den Ultraschallwandler gekoppelt ist,
wobei die Elektronikschaltung dafür konfiguriert ist, eine Signalverarbeitung umzusetzen, welche einen mit Flüssigkeit gefüllten Raum vor dem Ultraschallwandler erkennt,
**dadurch gekennzeichnet, dass**
die Elektronikschaltung weiterhin dafür konfiguriert ist, eine Zeit-Dehn-Technik umzusetzen, welche Informationen von aus zu dem Ultraschallwandler zurückkehrenden Echos zur Präsentation an einen Nutzer formatiert.

2. Vorrichtung nach Anspruch 1, welche weiterhin eine optische Anzeige aufweist.

3. Vorrichtung nach Anspruch 1, wobei mindestens ein Teil der Elektronikschaltung innerhalb der Längsbohrung der Sonde angeordnet ist.

4. Vorrichtung nach Anspruch 1, wobei mindestens ein Teil der Elektronikschaltung in einem Gehäuse angeordnet ist, welches über ein flexibles Kabel vernetzbar mit dem Ultraschallwandler verknüpft ist.

5. Vorrichtung nach Anspruch 1, wobei die Elektronikschaltung in zwei Teilen umgesetzt ist, welche durch ein flexibles Kabel vernetzbar verknüpft sind.

6. Vorrichtung nach Anspruch 1, wobei ein erster Teil der Elektronikschaltung innerhalb eines ersten Gehäuses angeordnet ist, welches mechanisch mit der Sonde gekoppelt ist, und ein zweiter Teil der Elektronikschaltung innerhalb eines zweiten Gehäuses angeordnet ist und mit dem ersten Teil über ein flexibles Kabel vernetzbar verknüpft ist.

7. Vorrichtung nach Anspruch 1, wobei ein erster Teil der Elektronikschaltung innerhalb eines ersten Gehäuses angeordnet ist, welches mechanisch mit der Sonde gekoppelt ist, und ein zweiter Teil der Elektronikschaltung innerhalb eines zweiten Gehäuses angeordnet ist und mit dem ersten Teil über eine kabellose Schnittstelle verbunden ist.

8. Vorrichtung nach Anspruch 1, wobei eine optische Anzeige innerhalb eines Gehäuses angeordnet ist, welches mechanisch mit der Sonde gekoppelt ist.

9. Vorrichtung nach Anspruch 4, wobei die Sonde und das Kabel als Einwegteile ausgestaltet sind, und die Elektronikschaltung und das Gehäuse als Mehrwegteile ausgestaltet sind.

10. Vorrichtung nach Anspruch 1, welche weiterhin einen Katheter aufweist, welcher ein proximales Ende und ein distales Ende mit einer Längsbohrung hierdurch aufweist, und wobei die Sonde so dimensioniert und dafür ausgelegt ist, dass sie gezielt in der Längsbohrung des Katheters angeordnet werden kann.

11. Vorrichtung nach Anspruch 1, wobei der Ultraschallwandler innerhalb der Längsbohrung der Sonde angeordnet ist.

12. Vorrichtung nach Anspruch 1, wobei der Ultraschallwandler außerhalb der Längsbohrung der Sonde angeordnet ist.

13. Vorrichtung nach Anspruch 10, wobei der Katheter eine innere Lippe beinhaltet, gegen welche mindestens ein Teil der Sonde gedrückt wird.

14. Vorrichtung nach Anspruch 10, wobei der Katheter ein offenes distales Ende aufnimmt.

15. Vorrichtung nach Anspruch 10, wobei der Katheter mindestens eine Auslassöffnung in einer Seite des Katheters aufnimmt.

16. Vorrichtung nach Anspruch 1, wobei die Sonde aus einem elektrisch leitfähigen Material besteht, und dazu verwendet wird, einen Teil einer elektrischen Verbindung zu dem Ultraschallwandler zu bilden.

## Revendications

1. Appareil chirurgical, comprenant :
un stylet ayant une extrémité proximale et une extrémité distale et muni d'un alésage longitudinal à travers celui-ci ;
un transducteur à ultrasons disposé au niveau de l'extrémité distale dudit alésage longitudinal dudit stylet ; et
un circuit électronique couplé audit transducteur à ultrasons ;
dans lequel ledit circuit électronique est configuré pour implémenter un traitement de signal qui détecte un espace rempli de fluide en face dudit transducteur à ultrasons,
**caractérisé en ce que**
ledit circuit électronique est en outre configuré pour implémenter une technique d'étirement du temps qui formate des informations à partir d'échos revenant vers le transducteur à ultrasons pour une présentation à un utilisateur.

2. Appareil selon la revendication 1, comprenant en outre un affichage visuel.

3. Appareil selon la revendication 1, dans lequel au moins une partie dudit circuit électronique est disposée dans l'alésage longitudinal dudit stylet.

4. Appareil selon la revendication 1, dans lequel au moins une partie dudit circuit électronique est contenue dans un boîtier qui est relié de manière connectable audit transducteur à ultrasons par l'intermédiaire d'un câble flexible.

5. Appareil selon la revendication 1, dans lequel ledit circuit électronique est implémenté en deux parties qui sont reliées de manière connectable par l'intermédiaire d'un câble flexible.

6. Appareil selon la revendication 1, dans lequel une première partie dudit circuit électronique est disposée dans un premier boîtier qui est couplé mécaniquement audit stylet, et une seconde partie dudit circuit électronique est disposée dans un second boîtier et est reliée de manière connectable à la première partie par l'intermédiaire d'un câble flexible.

7. Appareil selon la revendication 1, dans lequel une première partie dudit circuit électronique est disposée dans un premier boîtier qui est couplé mécaniquement audit stylet, et une seconde partie dudit circuit électronique est disposée dans un second boîtier et est reliée à la première partie par l'intermédiaire d'une interface sans fil.

8. Appareil selon la revendication 1, dans lequel un affichage visuel est disposé dans un boîtier qui est couplé mécaniquement audit stylet.

9. Appareil selon la revendication 4, dans lequel ledit stylet et ledit câble sont configurés sous forme de parties à usage unique, et ledit circuit électronique et ledit boîtier sont configurés sous forme de parties à usage multiple.

10. Appareil selon la revendication 1, comprenant en outre un cathéter ayant une extrémité proximale et une extrémité distale muni d'un alésage longitudinal à travers celui-ci et ledit stylet étant dimensionné et adapté pour une disposition sélective dans ledit alésage longitudinal dudit cathéter.

11. Appareil selon la revendication 1, dans lequel ledit transducteur à ultrasons est disposé dans l'alésage longitudinal dudit stylet.

12. Appareil selon la revendication 1, dans lequel ledit transducteur à ultrasons est disposé à l'extérieur de l'alésage longitudinal dudit stylet.

13. Appareil selon la revendication 10, dans lequel ledit cathéter incorpore une lèvre interne contre laquelle au moins une partie dudit stylet est appuyée.

14. Appareil selon la revendication 10, dans lequel ledit cathéter incorpore une extrémité distale ouverte.

15. Appareil selon la revendication 10, dans lequel ledit cathéter incorpore au moins un orifice de drainage dans un côté du cathéter.

16. Appareil selon la revendication 1, dans lequel ledit stylet est composé d'un matériau électriquement conducteur, et est utilisé pour former une partie d'une connexion électrique audit transducteur à ultrasons.
